# FASCICULE DE BREVET EUROPEEN

(11) **EP 0 494 568 B1**
(45) Date de publication et mention de la délivrance du brevet: **17.05.1995**
(21) Numéro de dépôt: 91403571.2
(22) Date de dépôt: 31.12.1991
(51) Int. Cl.: C07C 209/36, C07C 211/52, B01J 23/62

(54) **Réduction catalytique de composés nitroaromatiques chlorés en anilines chlorées**
Katalytische Reduktion von chlorierten nitroaromatischen Verbindungen in chlorierte Aniline
Catalytic reduction of chlorinated nitroaromatic compounds to chlorinated anilines

(30) Priorité: 07.01.1991 FR 9100132
(43) Date de publication de la demande: 15.07.1992
(73) Titulaire: INSTITUT FRANCAIS DU PETROLE, 92502 Rueil-Malmaison (FR)
(72) Inventeur: Didillon, Blaise, F-92500 Rueil Malmaison (FR); Le Peltier, Fabienne, F-92500 Rueil Malmaison (FR); Candy, Jean-Pierre, F-69300 Caluire (FR); Sarrazin, Patrick, F-92500 Rueil Malmaison (FR); Boitiaux, Jean-Paul, F-78300 Poissy (FR); Basset, Jean-Marie, F-69100 Villeurbanne (FR)

(56) Documents cités:
- EP-A- 0 011 090
- EP-A- 0 325 892
- DE-A- 2 615 079
- DE-A- 3 019 582
- DE-B- 1 233 878
- DE-B- 1 518 402
- CHEMICAL ABSTRACTS, vol. 98, no. 13, 28 mars 1983, page 569, abrégé no. 106948d, Columbus, Ohio, US; SU-A-1950718 (A.I. GEL'BSHTEIN et al.) 15.08.1982

## Description

L'invention concerne un procédé de production d'aniline substituée par hydrogénation du composé nitroaromatique correspondant.

Ces composés nitroaromatiques répondent aux formules générales suivantes (I) ou (II) : 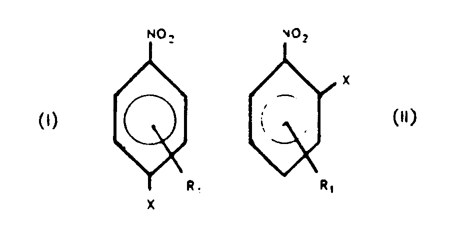
où X est un halogène et R₁ est l'hydrogène ou un substituant du noyau aromatique choisi parmi : les alkyls, les halogènes, les alkényles, le sulfoxy, le nitro, le nitroso, les amines, l'hydroxyle, les éthoxys et les acétoxys.

L'hydrogénation sélective en présence d'hydrogène de la ou des fonctions nitro portées pu le noyau aromatique conduit à la formation d'amines ayant la formule brute suivante (III) ou (IV) : 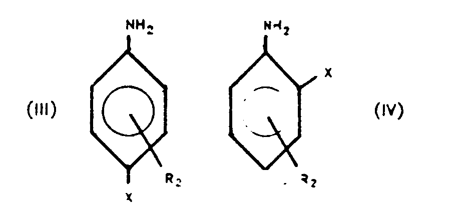
où X est un halogène et R₂ est l'hydrogène ou un substituant du noyau aromatique choisi parmi : les alkyles, les halogènes, les alkényls, le sulfoxy, les amines, l'hydroxyle, les éthoxys et les acétoxys.

Ces produits sont utilisés dans de nombreux secteurs industriels et on peut citer notamment la chimie des colorants.

Un des inconvénients rencontré dans la mise en oeuvre de cette réaction est qu'une partie des produits de la réaction subit une hydrogénolyse de la ou des liaisons carbone halogène X qui conduit aux produits secondaires suivants de formule (V) ou (VI) : 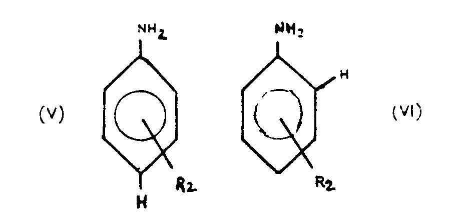
où R est l'hydrogène ou un substituant du noyau aromatique choisi parmi : les alkyles, les halogènes, les alkényls, le sulfoxy, les amines, l'hydroxyle, les éthoxys et les acétoxys.

De plus, qu'il y ait ou pas de substituant autre que le groupement nitro sur le noyau aromatique, il a été observé que la décomposition d'intermédiaires de réaction partiellement réduits de type hydroxylamine, azoxy, azo ou hydrazo mène à la formation de sous-produits lourds (KIRK-OTHMER Encycl. chem. Technol. 3th Ed., Vol.2, 355) qui diminuent le rendement global de la réaction et provoquent une consommation excessive de catalyseur.

L'obtention de conversions élevées, si possible totales, avec la meilleure sélectivité possible est donc une caractéristique difficile à obtenir pour cette transformation.

Les métaux du groupe VIII sont connus pour catalyser la réduction des composés nitroaromatiques sous pression d'hydrogène. Ils fonctionnent avantageusement en phase liquide, c'est à dire en présence d'un solvant mais l'obtention de sélectivités intéressantes n'est possible qu'avec des ajouts. Ainsi l'hydrogénation sélective de composés aromatiques nitrochlorés a été décrite en présence d'un catalyseur au platine sulfuré supporté sur charbon (DE-B-1,959,578) et sur d'autres catalyseurs à base de métaux du groupe VIII sulfurés (US-A-3,350,450). Ces métaux sulfurés sont généralement peu actifs et l'on préfère parfois ajouter l'agent sélectivant dans le milieu réactionnel. C'est ainsi que l'utilisation d'inhibiteurs tels que la triphénylphosphite (US-A-3,474,144) ou la morpholine (US-A-3,145,231) en présence de catalyseur à base de platine permettent de limiter fortement la déhalogènation des produits de la réaction. Plus récemment, le brevet EP-B-325,892 utilisant des sels de formamidine comme inhibiteur de la réaction de déhalogènation en présence de catalyseur à base de nickel de Raney revendique l'obtention de sélectivités élevées.

La transformation de ces composés nitroaromatiques fait l'objet d'un certain nombre de publications. L'utilisation de borure de nickel à la place de nickel de Raney comme catalyseur semble limiter les intermédiaires de réaction responsables de la formation de sous produits lourds (Ind. Eng. Chem. Prod. Res. Dev., 21, 279-281, 1982). Le même catalyseur en présence de l'acétate de formamidine dans le méthanol permet l'obtention d'une sélectivité de 99,4 % pour le 1-chloro 2-nitrobenzène (Heterogeneous catalysis and fine chemicals, 2nd International Symposium, Guisnet et al., Elsevier to be published).

EP-A-11.090 et DE-A-3.019.582 concernent un procédé d'hydrogénation catalytique de composés nitroaromatiques utilisant un catalyseur comportant un support, un métal noble et un ou plusieurs composés des groupes IVa, Va et VIa du tableau périodique. Le problème que tente de résoudre la présente invention est de sélectionner un catalyseur particulier permettant de réduire la déhalogénation constatée lors de l'hydrogénation de dérivés halogénonitroaromatiques.

Il a été découvert dans la présente invention qu'il est possible de réaliser l'hydrogénation de composés nitroaromatiques substitués ou non avec des sélectivités très élevées en composés aminoaromatiques correspondants sans diminution de l'activité du métal de base, voire avec promotion et ceci sans additif dans le milieu réactionnel. On opère dans un réacteur continu ou discontinu en présence d'hydrogène sous une pression totale généralement comprise entre 10 et 100 bars (1 et 10 Mégapascals) et de préférence entre 20 et 80 bars (2 et 8 Mégapascals), bien que l'on puisse opérer sans inconvénient, par exemple, jusqu'à 300 bars (30 Mégapascals), à une température généralement comprise entre 0 et 100 degrés celsius et de préférence entre 30 et 80 degrés celsius en présence d'au moins un catalyseur métallique supporté (catalyse hétérogène) renfermant (a) un métal du groupe VIII qui est le rhodium et dont le pourcentage pondéral par rapport à la masse totale du catalyseur est de préférence choisi entre 0,1 et 10 % exprimé en métal et plus particulièrement entre 0,5 et 5 % et (b) un élément additionnel métallique du groupe IV.A qui est l'étain, dont le pourcentage pondéral est choisi de préférence entre 0,01 et 10 % exprimé en métal et plus particulièrement entre 0,1 et 5 %. Le rapport molaire élément métallique du groupe IV sur métal du groupe VIII est avantageusement compris entre 0,8 et 3 et de préférence entre 0,9 et 2,6. Le support peut être choisi dans le groupe constitué par la silice, les différents types d'alumine, les silice-alumines, les aluminates des éléments des groupes I.A, II.A ou II.B de la classification périodique des éléments comme par exemple les aluminates de Ca, Mg, Ba, Zn, Na, K, Cd et les aluminates mixtes, et le charbon et de préférence dans le groupe constitué par la silice et les aluminates de métaux alcalins et/ou alcalino-terreux et/ou de zinc et/ou de cadmium et les aluminates mixtes.

Le catalyseur peut être préparé par différentes procédures d'imprégnation du support. L'opération d'imprégnation consiste, par exemple, à mettre en contact le support préformé et une solution aqueuse ou organique d'un composé du métal ou des métaux choisi(s), le volume de solution étant de préférence en excès par rapport au volume de rétention du support ou égal à ce volume. Après avoir laissé le contact entre le support et la solution pendant plusieurs heures, le support imprégné est filtré, lavé à l'eau distillé, séché et calciné sous air habituellement entre 110°C et 600°C et de préférence entre 110°C et 500°C. Avant utilisation, on réduit le catalyseur sous hydrogène habituellement entre 50°C et 600°C et de préférence entre 90°C et 500°C, cette réduction pouvant être effectuée aussitôt après calcination, ou plus tard chez l'utilisateur. L'étain peut être introduit en solution aqueuse ou en solution hydrocarbonée selon la nature du précurseur utilisé.

D'une manière préférée le catalyseur est obtenu par imprégnation du support, à l'aide d'une solution aqueuse ou organique du composé de métal du groupe VIII, le volume de solution étant de préférence en excès par rapport au volume de rétention du support ou égal à ce volume. Le support imprégné est ensuite filtré, éventuellement lavé à l'eau distillé puis séché et calciné sous air habituellement entre environ 110°C et environ 600°C, et de préférence entre environ 110°C et environ 500°C, puis ensuite réduit sous hydrogène à une température habituellement comprise entre environ 50°C et environ 600°C et de préférence entre environ 80°C et environ 500°C ; le produit obtenu est alors imprégné par une solution aqueuse ou organique d'un composé d'étain.

D'une manière particulièrement avantageuse, on utilise une solution d'un hydrocarbyl-étain dans un hydrocarbure saturé, selon la technologie décrite dans le brevet US-A-4,548,918 de la demanderesse.

Parmi les solvants organiques utilisables pour la préparation du catalyseur on peut citer à titre d'exemples non limitatifs les hydrocarbures, les hydrocarbures halogènés, les cétones et les éthers. L'emploi d'un solvant n'est pas indispenssable lorsque le composé d'étain est lui-même liquide comme cela est, par exemple, le cas pour le tétrabutyl-étain.

Une autre méthode consiste à malaxer la poudre humide de support avec les précurseurs du catalyseur et à mettre ensuite en forme et sécher.

Les exemples des précurseurs métalliques utilisables dans la préparation du catalyseur sont les suivants :
Pour le métal du groupe VIII, on peut utiliser des composés tels que les chlorures, les nitrates, les composés halogéno-aminés, les composés aminés, les sels d'acides organiques solubles dans le solvant d'imprégnation.

On peut aussi utiliser des composés organométalliques du métal du groupe VIII en solution dans un solvant organique, par exemple un hydrocarbure. Comme exemple d'hydrocarbures on peut citer les hydrocarbures paraffiniques saturés dont la cha'ne hydrocarbonée renferme de 6 à 12 atomes de carbone par molécule, les hydrocarbures naphténiques qui renferment de 6 à 12 atomes de carbone par molécule ou encore les hydrocarbures aromatiques renfermant de 6 à 12 atomes de carbone par molécule. A titre d'exemples de composés organométalliques de métal du groupe VIII on peut citer : les composés carbonyles, halogénocarbonyles et les acétylacétonates sans que cette liste soit limitative.

L'étain peut être introduit de préférence sous la forme d'au moins un composé organique choisi dans le groupe formé par les complexes, en particulier les complexes polycétoniques, du métal du groupe IV.A et les hydrocarbylmétaux tels que les alkyles, les cycloalkyles, les aryles, les alkylaryles et les arylalkyles métaux.

L'introduction du métal IV.A est avantageusement effectuée à l'aide d'une solution dans un solvant organique du composé organométallique dudit métal IV.A. On peut également employer des organohalogénés des métaux IV.A. Comme composés de métaux IV.A on citera en particulier le tetrabutyl-étain, le tetraméthyl-étain, le tetrapropyl-étain, le diphényl-étain.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer le n-heptane, le méthylcyclohexane, le toluène et le chloroforme. On peut utiliser des mélanges des solvants définis ci-dessus.

L'étain peut aussi être introduit par l'intermédiaire de composés tels que les chlorures, les bromures et le nitrate d'étain, en solution aqueuse ou organique.

Le support peut être de nature variée, comme déjà mentionné plus haut. Un support particulièrement adapté possède des caractéristiques spécifiques telles qu'une aire spécifique, déterminée par la méthode B.E.T., comprise entre 10 et 500 m² par gramme et de préférence comprise entre 50 et 500 m² par gramme et un volume poreux total de 0,2 à 1,3 cm³ par gramme de support.

Une fois les métaux fixés sur le support, le catalyseur subit avantageusement un traitement d'activation sous hydrogène à haute température, par exemple 50-600°C, afin d'obtenir une phase métallique active. La procédure de ce traitement sous hydrogène consiste par exemple en une montée lente de la température sous courant d'hydrogène jusqu'à la température maximale de réduction, comprise par exemple entre 50°C et 600°C et de préférence entre 80°C et 500°C, suivie d'un maintien pendant par exemple 1 à 6 heures à cette température.

Les exemples suivants, non limitatifs, illustrent l'invention.

La préparation du catalyseur se fait par imprégnation de chlorure de rhodium chloropentamine en solution ammoniacale sur une silice dont la surface spécifique est égale 280 m² par gramme et le volume poreux total est égal 80 cm³ par 100 grammes, suivie d'une filtration, d'une calcination sous air 450°C et d'une réduction sous hydrogène à 450°C.

Le catalyseur fini contient 1 % de rhodium et sera appelé catalyseur A.

### EXEMPLE 1 (COMPARATIF). On se propose ici de réduire du 1-chloro 4-nitrobenzène.

Le catalyseur A est chargé dans un réacteur de type Grignard contenant un solvant organique, le normal heptane. Le réacteur est ensuite fermé et purgé. La pression d'hydrogène est augmentée jusqu'à 40 bars (4 Mégapascals) et la température élevée jusqu'à 96°C. Ces conditions sont maintenues pendant 20 minutes sous agitation.

La pression et la température sont ensuite ramenées respectivement à 20 bars (2 Mégapascals) et à 30°C. Une solution de 1-chloro 4-nitrobenzène dans le n-heptane est alors injectée dans le réacteur dans une proportion de 200 moles de 1-chloro 4-nitrobenzène par atome gramme de rhodium.

L'évolution de la composition du milieu réactionnel est suivie par chromatographie en phase gazeuse.

Les résultats obtenus sont présentés dans le tableau 1.

**Tableau 1**

| Temps (h) | Conversion (%) | Sélectivité (%) |
|---|---|---|
| 0 | 0 | - |
| 0,5 | 7 | 0 |
| 1 | 10 | 0 |
| 1,5 | 14 | 0 |
| 3 | 26 | 0 |
| 4 | 38 | 6 |
| 20 | 100 | 21 |

Le rendement obtenu en 1-chloro 4-aminobenzène est de 21 % après 20 heures. Il y a donc une déchloration importante du substrat lors de la réaction.

### EXEMPLE 2 (SELON L'INVENTION)

Le catalyseur A est chargé dans un réacteur de type Grignard contenant un solvant organique, le normal heptane. Du tétrabutylétain est alors injecté dans le solvant. Le réacteur est ensuite fermé et purgé. La pression d'hydrogène est augmentée jusqu'à 40 bars (4 Mégapascals) et la température élevée jusqu'à 96°C. Ces conditions sont maintenues pendant 20 minutes sous agitation.

Le catalyseur fini présente un rapport atomique étain sur rhodium de 2. % Rh : 1 % ; % Sn : 2,2 % (en poids). On l'appelle catalyseur B.

La pression et la température sont ensuite ramenées respectivement à 20 bars (2 Mégapascals) et à 30°C. Une solution de 1-chloro 4-nitrobenzène dans le n-heptane est alors injectée dans le réacteur dans une proportion de 200 moles de 1-chloro 4-nitrobenzène par atome gramme de rhodium, comme dans l'exemple 1.

L'évolution de la composition du milieu réactionnel est suivie par chromatographie en phase gazeuse.

Les résultats obtenus sont présentés dans le tableau 2.

**Tableau 2**

| Temps (h) | Conversion (%) | Sélectivité (%) |
|---|---|---|
| 0 | 0 | - |
| 0,5 | 34 | 13 |
| 1,5 | 82 | 88 |
| 3 | 100 | 99,7 |
| 4 | 100 | 97 |
| 20 | 100 | 64 |

Le rendement maximum en 1-chloro 4-aminobenzène obtenu est de 99,7 % après 3 heures. La déchloration du substrat est donc très faible. D'autre part, le temps au bout duquel 38 % du 1-chloro 4-nitrobenzène sont convertis est sept fois plus court que dans l'exemple 1.

### EXEMPLE 3 (SELON L'INVENTION). Réduction du 1-chloro 2-nitrobenzène.

Le catalyseur A est chargé dans un réacteur de type Grignard contenant un solvant organique, le normal heptane. Du tétrabutylétain est alors injecté dans le solvant. Le réacteur est ensuite fermé et purgé. La pression d'hydrogène est augmentée jusqu'à 40 bars (4 Mégapascals) et la température élevée jusqu'à 96°C. Ces conditions sont maintenues pendant 20 minutes sous agitation.

Le catalyseur fini présente un rapport atomique étain sur rhodium de 2. % Rh : 1 ; % Sn : 2,2 %. C'est le catalyseur 3 déjà utilisé à l'exemple précédent.

La pression et la température sont ensuite ramenées respectivement à 20 bars (2 Mégapascals) et à 30°C. Une solution de 1-chloro 2-nitrobenzène dans le n-heptane est alors injectée dans le réacteur dans une proportion de 200 moles de 1-chloro 2-nitrobenzène par atome gramme de rhodium.

L'évolution de la composition du milieu réactionnel est suivie par chromatographie en phase gazeuse.

Les résultats obtenus sont présentés dans le tableau 3.

**Tableau 3**

| Temps (h) | Conversion (%) | Sélectivité (%) |
|---|---|---|
| 0 | 0 | - |
| 1 | 7 | 57 |
| 1,5 | 11 | 67 |
| 2 | 19 | 66 |
| 2,5 | 22 | 84 |
| 3 | 26 | 100 |
| 4 | 39 | 100 |
| 20 | 98 | 100 |

Le rendement maximum en 1-chloro 2-aminobenzène obtenu dans cet exemple est de 98 %. La tendance à inhiber la déchloration en présence du catalyseur B, observée dans l'exemple 2 se confirme ici avec l'absence totale de déchloration.

## Revendications

1. Procédé de production d'un composé aminoaromatique répondant à l'une des formules (III) ou (IV). 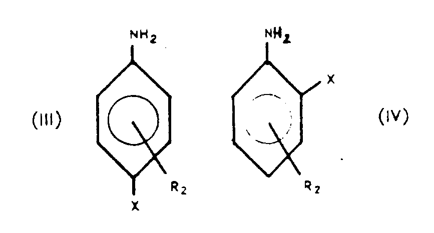 où X est un halogène et R₂ est choisi parmi l'hydrogène et un substituant du noyau aromatique choisi dans le groupe constitué par les radicaux alkyles, les halogènes, les radicaux alkényles, les radicaux sulfoxy, les radicaux aminés, l'hydroxyle, les éthoxys et les acétoxys, par hydrogénation sélective en présence d'hydrogène d'au moins une fonction nitro porté par le noyau aromatique d'un composé nitroaromatique répondant à la formule (I) ou (II). 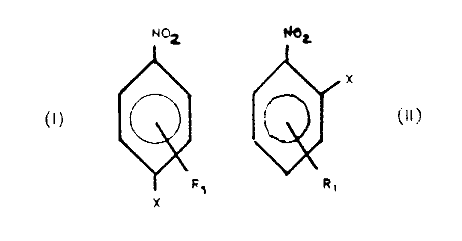 où X est un halogène et R₁ est choisi parmi l'hydrogène et un substituant du noyau aromatique choisi dans le groupe constitué par les radicaux alkyles, les halogènes, les radicaux alkényles, les radicaux sulfoxy, nitro, nitroso, amines, l'hydroxyle, les éthoxys et les acétoxys, procédé caractérisé en ce que l'on opère en présence d'un catalyseur renfermant un support et renfermant :
(a) un métal du groupe VIII, le rhodium, (b) un élément additionnel métallique du groupe IV.A, l'étain.

2. Procédé selon la revendication 1 dans lequel le pourcentage pondéral (exprimé en métal) par rapport à la masse totale du catalyseur en métal du groupe VIII est comprise entre 0,1 et 10 %.

3. Procédé selon la revendication 2 dans lequel ledit pourcentage est compris entre 0,5 et 5 %.

4. Procédé selon l'une des revendications 1 à 3 dans lequel le pourcentage pondéral (exprimé en métal) du métal du groupe IV.A, par rapport à la masse totale du catalyseur, est comprise entre 0,01 et 10 %.

5. Procédé selon la revendication 4 dans lequel ledit pourcentage est compris entre 0,1 et 5 %.

6. Procédé selon l'une des revendications 1 à 5 dans lequel le rapport molaire élément métallique du groupe IV sur métal du groupe VIII est compris entre 0,8 et 3.

7. Procédé selon la revendication 6 dans lequel ledit rapport est compris entre 0,9 et 2,6.

8. Procédé selon l'une des revendications 1 à 7 dans lequel le catalyseur renferme au moins 2 métaux du groupe IV.A.

## Claims

1. A process for the production of an aminoaromatic compound corresponding to one of formulae (III) or (IV) 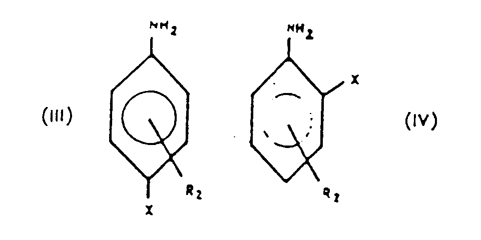 where X is a halogen and R₂ is selected from hydrogen and a substituent of the aromatic ring selected from the group constituted by alkyl radicals, halogens, alkenyl radicals, sulfoxy radicals, amino radicals, hydroxyl, ethoxys and acetoxys, by selective hydrogenation in the presence of hydrogen of at least one nitro function carried by the aromatic ring of a nitroaromatic compound corresponding to Formula (I) or (II) 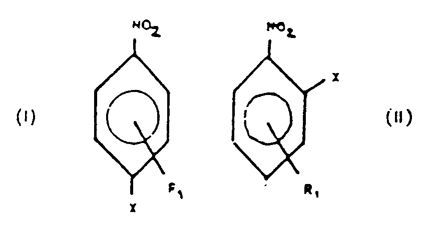 where X is a halogen and R₁ is selected from hydrogen and a substituent of the aromatic ring selected from the group constituted by alkyl radicals, halogens, alkenyl radicals, sulfoxy radicals, nitro, nitroso, amines, hydroxyl, ethoxys and acetoxys, which process is characterised in that the operation is carried out in the presence of a catalyst containing a carrier and containing:
(a) a group VIII metal, rhodium, (b) one additional metal element from group IV.A, tin.

2. A process according to Claim 1, wherein the percentage by weight (expressed in metal) in relation to the total mass of Group VIII metal in the catalyst is between 0.1 and 10%.

3. A process according to Claim 2, wherein said percentage is between 0.5 and 5%.

4. A process according to one of Claims 1 to 3, wherein the percentage by weight (expressed in metal) of the group IV.A metal in relation to the total mass of catalyst is between 0.01 and 10%.

5. A process according to Claim 4, wherein said percentage is between 0.1 and 5%.

6. A process according to one of Claims 1 to 5, wherein the molar ratio of group IV metal element to group VIII metal is between 0.8 and 3.

7. A process according to Claim 6, wherein said ratio is between 0.9 and 2.6.

8. A process according to one of Claims 1 to 7, wherein the catalyst contains at least 2 group IV.A metals.

## Patentansprüche

1. Verfahren zur Herstellung einer aromatischen Aminverbindung, die einer der Formeln (III) oder (IV) 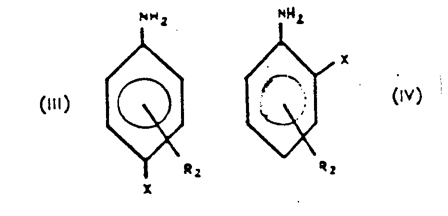 entspricht, in denen X ein Halogenatom ist und R₂ aus einem Wasserstoffatom und einem Substituenten mit aromatischem Kern ausgewählt ist, der aus der Gruppe ausgewählt ist, die aus den Alkylresten, den Halogenen, den Alkenresten, den Sulfoxyresten, den aminierten Resten, dem Hydroxylrest, den Ethoxy- und den Acetoxyresten besteht, durch selektive Hydrierung in Gegenwart von Wasserstoff wenigstens einer durch den aromatischen Kern einer nitroaromatischen Verbindung getragenen Nitrofunktion, die der Formel (I) oder (II) 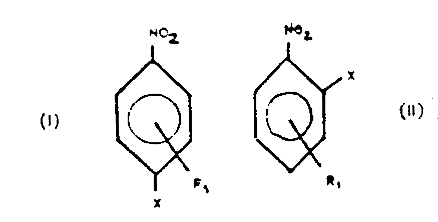 entspricht, in denen X ein Halogenatom ist und R₁ aus einem Wasserstoffatom und einem Substituenten mit aromatischem Kern ausgewält ist, der aus der Gruppe ausgewählt ist, der aus den Alkylresten, den Halogenen, den Alkenresten, den Sulfoxy-, Nitro-, Nitroso-, Aminresten, dem Hydroxylrest, den Ethoxy- und Acetoxyresten besteht, wobei das Verfahren dadurch gekennzeichnet ist, daß man in Gegenwart eines Katalysators arbeitet, der einen Träger umfaßt und:
(a) ein Metall der Gruppe VIII des Periodensystems, das Rhodium,
(b) ein zusätzliches metallisches Element der Gruppe IV A des Periodensystems, das Zinn,
umfaßt.

2. Verfahren nach Anspruch 1, worin der Massenanteil (bezogen auf Metall), bezogen auf die Gesamtmasse an Metall der Gruppe VIII des Periodensystems des Katalysators, zwischen einschließlich 0,1 bis 10 % liegt.

3. Verfahren nach Anspruch 2, wobei der Massenanteil zwischen einschließlich 0,5 und 5 % liegt.

4. Verfahren nach einem der Ansprüche 1 bis 3, worin der Massenanteil (bezogen auf Metall) des Metalls der Gruppe IV A des Periodensystems, bezogen auf die Gesamtmasse des Katalysators, zwischen einschließlich 0,01 und 10 % liegt.

5. Verfahren nach Anspruch 4, worin der Massenanteil zwischen einschließlich 0,1 und 5 % liegt.

6. Verfahren nach einem der Ansprüche 1 bis 5, worin das Molverhältnis von metallischem Element der Gruppe IV des Periodensystems über Metall der Gruppe VIII des Periodensystems zwischen einschließlich 0,8 und 3 liegt.

7. Verfahren nach Anspruch 6, worin das Verhältnis zwischen einschließlich 0,9 und 2,6 liegt.

8. Verfahren nach einem der Ansprüche 1 bis 7, worin der Katalysator wenigstens zwei Metalle der Gruppe IV A des Periodensystems umfaßt.
